# EUROPÄISCHE PATENTSCHRIFT

(11) **EP 2 114 486 B1**
(45) Veröffentlichungstag und Bekanntmachung des Hinweises auf die Patenterteilung: **22.02.2012**
(21) Anmeldenummer: 07817787.0
(22) Anmeldetag: 07.11.2007
(51) Int. Cl.: A61M 1/16

(54) **Medizintechnisches Gerät**
Medical apparatus
Appareil médical

(30) Priorität: 21.12.2006 DE 102006060611
(43) Veröffentlichungstag der Anmeldung: 11.11.2009
(73) Patentinhaber: Völker, Manfred, 63825 Blankenbach (DE); Becker, Franz Ferdinand, 63110 Rodgau (DE)
(72) Erfinder: BECKER, Franz Ferdinand, 63110 Rodgau (DE)
(74) Vertreter: Flosdorff, Jürgen
(86) Internationale Anmeldenummer: PCT/DE2007/001999
(87) Internationale Veröffentlichungsnummer: WO 2008/074282

(56) Entgegenhaltungen:
- WO-A-2004/062710
- US-A- 4 718 022
- US-A- 5 624 551
- US-A1- 2005 245 899

## Beschreibung

Die Erfindung betrifft ein medizintechnisches Gerät, das an eine Wasserleitung zur Zufuhr von Wasser oder aufbereitetem Wasser in das medizinische Gerät anschließbar ist

Die Erfindung betrifft allgemein eine Anschlußanordnung für medizintechnische Geräte, denen für ihren Betrieb Wasser oder aufbereitetes Wasser zugeführt werden muß. Insbesondere betrifft die Erfindung eine Anschlußanordnung für solche medizintechnischen Geräte, die im Online-Verfahren aus aufbereitetem Wasser und einem Konzentrat die für die Behandlung notwendige Anwendungslösung durch deren Mischungen herstellen, wie dies vor allem bei Dialysegeräten der Fall ist.

Die Erfindung wird nachfolgend im Zusammenhang mit Dialysegeräten beschrieben.

Der Anschluß eines solchen Gerätes kann als Einzelanschluß erfolgen oder aber als Anschluß an eine Ringleitung, an die mehrere Dialysegeräte angeschlossen werden können.

Die Flüssigkeitskomponenten, die bei einer Dialyse zum Einsatz kommen, müssen unbedingt rein sein, da Verunreinigungen in der Dialysierflüssigkeit den behandelten Patienten gefährden würden. Bakterien, Toxine und Pilzsporen in der Dialysierflüssigkeit können lebensgefährlich sein. Bei der überwachten industriellen Fertigung der Dialysekonzentrate, aus denen durch Mischen mit aufbereitetem Wasser bzw. Permeat im Dialysegerät die Dialysierflüssigkeit hergestellt wird, ist auszuschließen, daß die genannten Verunreinigungen in die Produkte gelangen. Wenn sie - was bisher leider in der Praxis anzutreffen ist - dennoch in der Dialysierflüssigkeit auftreten, muß dies bei der Herstellung der anwendungsfertigen Dialysierflüssigkeit geschehen.

Eine Hauptursache für die Verunreinigung der Dialysierflüssigkeit stellen nicht desinfizierte Leitungsabschnitte im oder am Dialysegerät dar. Hierzu zählt die Permeatzuleitung zwischen dem Dialysegerät und dem Permeat-Einzelanschluß oder der Permeatringleitung. Diese Permeatzuleitung wird bei den bisher bekannten Anschlußsystemen nicht in die Desinfektion des Dialysegerätes eingeschlossen, so daß sich hier gefährliche Verunreinigungen durch Bakterien, Toxine und Pilzsporen ausbilden und ansammeln können.

US-A-5 624 551 offenbart eine hydraulische Sicherheitsschaltung für ein Hämodialysegerät, das an eine oder mehrere Versorgungsringleitungen angeschlossen ist, durch die beispielsweise eine saure Lösung und eine Bikarbonat-Lösung zugeführt wird. Stromabwärts der Anschlussleitung des Gerätes sind zwei hintereinander angeordnete Absperrventile vorgesehen. Hinter dem zweiten Ventil zweigen von der weiterführenden Leitung zwei Leitungen ab, von denen eine zu einer Membranförderpumpe führt, die die Versorgungsflüssigkeit in ein nicht näher dargestelltes Rohrleitungssystem pumpt, während die andere Leitung sich weiter verzweigt. Bei dieser Anordnung geht es nicht um die Desinfektion eines bestimmten Bereichs des Gerätes, sondern darum, zu verhindern, dass bei Ausfall des zweiten Ventils und Unterdruck in der zugehörigen Leitung, Flüssigkeit aus dem Dialysegerät in die Ringleitung zurück fließt. Hierzu überwacht ein Drucksensor den Druck in dieser Leitung des Gerätes.

US-A-4 718 022 offenbart in Spate 9, Zeile 57, bis Spalte 10, Zeile 8, dass bei einem Desinfektionsvorgang der Konzentratauslass eines Dialysegerätes mit dem Wassereinlass verbunden wird und dass das Sterilisierungsmittel dann auch alle Bahnen des Leitungssystems zirkuliert. Dabei besteht die Gefahr, dass das Sterilisierungsmittel in die Wasserleitung zurück fließt, an die das Gerät angeschlossen ist.

Der vorliegenden Erfindung liegt die Aufgabe zugrunde, eine Anordnung zum Anschluß eines medizintechnischen Gerätes an eine Wasserversorgungsleitung anzugeben, bei der bzw, durch die die oben beschriebene Gefahr vermieden wird.

Diese Aufgabe wird erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, daß zum Anschluß des Gerätes ein Adapter verwendet wird, der einen ersten Zuleitungsabschnitt mit einem endseitigen Kupplungsteil zur Verbindung mit einem zugehörigen Kupplungsteil der Wasserleitung bzw. Permeatleitung, einem nachfolgenden Drucksensor und mit zwei nachfolgend hintereinander angeordneten Absperrventilen hat, wobei an dem anderen Ende des ersten Zuleitungsabschnitts ein Verzweigungsteil, bevorzugt ein Y-Leitungsstück angebracht ist, durch das der erste Zuleitungsabschnitt in zwei zweite Zuleitungsabschnitte aufgeteilt wird. Diese beiden zweiten Zuleitungsabschnitte sind derart mit zwei verschiedenen Leitungen des Gerätes verbunden, das bei einem Desinfektionsmodus des Gerätes Desinfektionsflüssigkeit die beiden zweiten Zuleitungsabschnitte durchströmt, während die beiden stellungsüberwachten Absperrventile geschlossen sind. Diese zweiten Zuleitungsabschnitte sind zweckmäßigerweise durch an das Y-Stück angebrachte Schläuche gebildet, die mit festen Schlauchanschlüssen, vorzugsweise Schlauchklemmen, an die beiden Leitungen des Gerätes angeschlossen sein können.

In weiteren Einzelheiten wird vorgeschlagen, daß einer der beiden zweiten Leitungsabschnitte mit der Wassereinlaßleitung des Gerätes verbunden ist, die zu dem Vorlaufbehälter für das Permeat führt, und daß der andere zweite Zuleitungsabschnitt mit einer anderen Leitung des Gerätes verbunden ist, die beim Desinfektionsvorgang von der Desinfektionsflüssigkeit durchströmt wird.

Durch diese Ausbildung werden bei einem Desinfektionsvorgang des Gerätes bei entsprechend betätigten Ventilen nicht nur die beiden zweiten Zuleitungsabschnitte des erfindungsgemäßen Anschlußsystems von der Desinfektionflüssigkeit durchströmt und damit in den Desinfektionskreislauf des Gerätes einbezogen, sondern dies trifft auch für die zu dem Vorlaufbehälter führende Wassereinlaßleitung des Gerätes zu, die bei einem herkömmlichen Gerät nicht in den Desinfektionskreislauf eingeschlossen ist.

Somit kann durch den erfindungsgemäßen Anschlußadapter der Zuleitungsbereich zwischen den beiden Absperrventilen und dem eigentlichen Gerät und außerdem die zu dem Vorlaufbehälter führende Permeateinlaßleitung bei jeder Desinfektion des Gerätes mit einbezogen werden, so daß diese bei den bisherigen Anschlußsystemen anzutreffende Gefahrenquelle für die Reinheit der Flüssigkeitskomponenten beseitigt ist.

Der zweite Zuleitungsabschnitt des Adapters, der nicht an die Wassereinlaßleitung des Gerätes angeschlossen ist, sondern an eine geeignete andere Leitung, kann an ein Zwischenleitungsstück angeschlossen sein, das mit dieser anderen Leitung verbunden ist und in dem ein Absperrventil angeordnet sein sollte. Durch das Absperrventil des Zwischenleitungsstücks kann die Zufuhr des Permeats - falls dies gewünscht wird - durch diese Leitung unterbunden werden, so daß die Permeatzufuhr nur durch den mit der Wassereinlaßleitung des Gerätes verbundenen zweiten Zuleitungsabschnitt erfolgt. Wenn das Absperrventil in dem Zwischenleitungsstück bei der Permeatzufuhr geöffnet bleibt, erfolgt die Permeatzufuhr durch beide zweite Leitungsabschnitte, was dann zweckmäßig sein kann, wenn auch diese andere Leitung in den Vorlaufbehälter des Gerätes einmündet. Bei einem Desinfektionsvorgang ist das Absperrventil in dem Zwischenleitungsstück geöffnet, damit die Desinfektionsflüssigkeit durch das Zwischenleitungsstück, den daran angeschlossenen zweiten Zuleitungsabschnitt und durch den anderen, an das Y-Stück anschließenden zweiten Zuleitungsabschnitt sowie durch die Wassereinlaßleitung strömen kann.

In näheren Einzelheiten wird vorgeschlagen, daß das Schnellkupplungsteil Bestandteil eines wasserdichten Gehäuses ist, in dem der Druckaufnehmer und die beiden Absperrventile untergebracht sind. Hierdurch ergibt sich ein kompakter Aufbau des erfindungsgemäßen Adapters.

Die Absperrventile sind bevorzugt stellungsüberwachte Magnetventile, die über das Gerät angesteuert werden. Im stromlosen Zustand sind die Magnetventile bevorzugt geschlossen. Dies ist bei der Betriebsphase "Desinfektion" der Fall. Vor jeder Behandlung und vor jeder Desinfektion des Gerätes wird die Stellung der beiden Absperrventile des Adapters automatisch überprüft.

Die beiden Magnetventile bilden ein redundantes System, das den Rückfluß von Desinfektionslösung in die Permeatringleitung (oder gegebenenfalls eine Einzelanschlußleitung) verhindert.

Weitere Einzelheiten und Merkmale der Erfindung ergeben sich aus der nachfolgenden Beschreibung bevorzugter Ausführungsformen sowie anhand der Zeichnungen. Dabei zeigen:
- Figur 1: eine rein schematische Darstellung eines Adapters und
- Figur 2: das Leitungssystem eines Dialysegerätes mit dem erfindungsgemäßen Anschlußsystem.

Der Adapter enthält ein Schnellkupplungsteil 1, das schnell und einfach mit einem zugehörigen Schnellkupplungsteil 2 einer Permeatleitung 3 koppelbar ist. Das Schnellkupplungsteil 1 ist am Ende eines ersten Zuleitungsabschnitt 4 angebracht, der nachfolgend einen Druckaufnehmer 20 und zwei hintereinander angeordnete 2/2-Wege-Ventile 5 und 6 enthält, mit denen der Durchfluß durch den ersten Zuleitungsabschnitt 4 wahlweise freigegeben oder abgesperrt werden kann. Die beiden 2/2-Wege-Magnetventile bilden ein redundantes Schutzsystem für die Permeatleitung 3.

An einen am anderen Ende des ersten Zuleitungsabschnitts 4 angebrachten Y-Verzweigungsteil 7 sind zwei zweite Zuleitungsabschnitte 8, 9 angeschlossen, die durch Schläuche gebildet sind.

Die Schnellkupplung 1,2 besteht aus Edelstahl. Die Stromversorgung und Steuerung der Magnetventile 5,6 erfolgt über das Dialysegerät. Im stromlosen Zustand sind die Magnetventile 5,6 geschlossen.

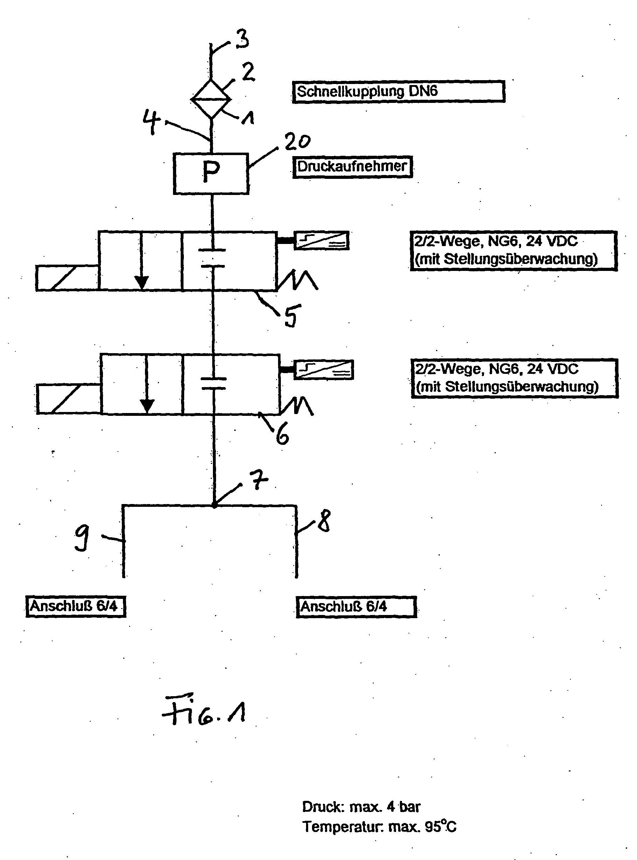

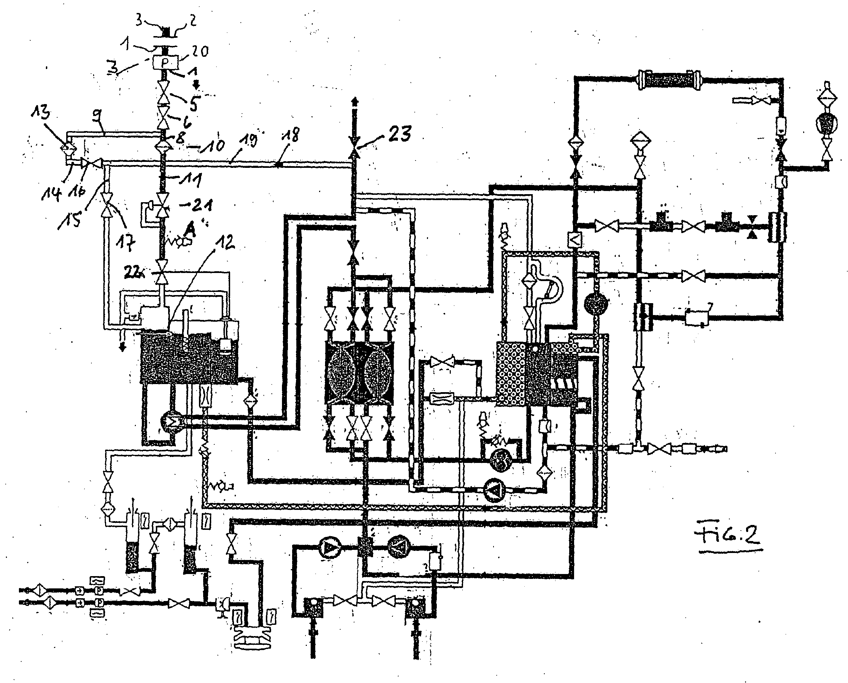

Figur 2 zeigt den Anschluß des Adapters gemäß Figur 1 an ein herkömmliches Dialysegerät. Der Zuleitungsabschnitt 8 ist über eine Schlauchklemme 10 mit der Permeateinlaßleitung 11 des Gerätes verbunden, die mit zwei aufeinander folgenden Ventilen 21 und 22 versehen ist und in den Vorlaufbehälter 12 des Gerätes einmündet.

Der andere zweite Zuleitungsabschnitt 9 ist über eine Schlauchklemme 13 an ein Zwischenleitungsstück 14 angeschlossen, das seinerseits mit einer Flüssigkeitsleitung 15 des Gerätes in Verbindung steht, die ebenfalls in den Vorlaufbehälter 12 einmündet.

Das Zwischenleitungsstück 14 enthält ein Absperrventil 16. In die Leitung 15 ist ebenfalls ein Absperrventil 17 eingeschaltet.

Das in das Zwischenstück 14 eingeschaltete Magnetventil 16 ist angeordnet, damit in dem Dialysegerät die bestehenden Funktionen nicht verändert werden. Durch Schließen des Ventils 16 bleibt in der Betriebsphase "Dialyse" der Permeateinlauf über den Anschluß 10 in den Vorlaufbehälter 12 für das Permeat erhalten.

In der Betriebsphase "Desinfektion" strömt Desinfektionsflüssigkeit in Richtung des Pfeils 18 durch die Leitung 19. Durch wechselseitiges Schalten der Ventile 16 und 17 zirkuliert die Desinfektionsflüssigkeit abwechselnd durch die Leitung 15 in den Vorlaufbehälter 12 und durch das Zwischenstück 14, die Zuleitungsabschnitte 9 und 8 sowie durch die Permeateinlaßleitung 11 in den Vorlaufbehälter 12. Die geschlossenen Magnetventile 5 und 6 verhindern den Rückfluß von Desinfektionsflüssigkeit in die Permeatringleitung.

Wenn das Magnetventil 16 in der Betriebsphase "Dialyse" geschlossen ist, ist der freie Einlauf des Permeats durch den Zuleitungsabschnitt 8 gewährleistet.

Beim Einsatz eines Dialysegerätes zur Durchführung einer extrakorporalen Dialysebehandlung unterscheidet man drei Betriebsphasen:
- Stand-by
- Dialyse
- Desinfektion

Diese drei Betriebsphasen werden nachfolgend im Zusammenhang mit der vorliegenden Erfindung in näheren Einzelheiten beschrieben.

### Stand-by

In diesem Betriebszustand sind die beiden Ventile 5 und 6 geschlossen. In den Leitungen zwischen dem Adapter und dem Dialysegerät befindet sich Permeat von der letzten Freispülung des Dialysegerätes. Somit besteht keine Gefährdung für Patienten, die an anderen Dialyseplätzen, an der gleichen Permeatleitung dialysiert werden.

### Dialyse

Die Betriebsphase Dialyse beginnt mit dem Test T₀ des Dialysegerätes. In diesen Test werden die Funktionselementes des Adapters mit einbezogen. Hierbei werden die Ventile 5 und 6 auf Funktion geprüft, in dem die Ventilstellungen nacheinander abgefragt werden. Hierbei erfolgt auch die Prüfung auf Dichtigkeit. Der Druck-Sensor 20 wird ebenfalls geprüft, indem eine Staudruck- und Fließdruckmessung erfolgt. Der gemessene Fließdruck wird mit der Messung des reduzierten Wassereingangsdrucks bei A (figur 2) hinter dem Ventil 21 verglichen. Unter Berücksichtigung einer entsprechenden Toleranz müssen die beiden Druckwerte übereinstimmen. Ist die Abweichung zu groß, wird ein Fehler angezeigt. Liegen die beiden Druckwerte beieinander wird der T₀-Test weitergeführt. Sollte bei dieser Messung der Fließdruck unterhalb des gewünschten Eingangsdrucks des Dialysegerätes liegen, wird ein Wassermangelalarm über das Ventil 21 ausgelöst. Damit kann das Dialysegerät nicht den T₀-Test beenden und geht somit nicht in die eigentliche Betriebsphase Dialyse. Ist der Fließdruck ausreichend, wird der T₀-Test fortgesetzt. Nach abgeschlossenem T₀-Test werden die Ventile 5 und 6 geöffnet. Das Ventil 16 ist geschlossen. Nach Öffnen der Ventile 21 und 22 fließt das Permeat in den Vorlaufbehälter. Somit ist der freie Einlauf in der Betriebsphase Dialyse weiterhin gewährleistet.

### Desinfektion

Nach der Dialyse erfolgt in der Regel eine Desinfektion des Dialysegerätes. Durch den Einsatz des Adapters soll bei jeder Desinfektion die Permeatleitung mit einbezogen werden. Es werden bei dem Dialysegerät die Desinfektionsarten thermische Desinfektion, chemo-thermische und chemisch Desinfektion unterschieden. Als Risiko wird für alle aufgeführten Desinfektionsarten angesehen, dass während oder nach der Desinfektion eine Rückströmung von Desinfektionslösung über den Adapter in die Permeatleitung erfolgen könnte. Dies ist möglich, wenn der Druck im Dialysegerät beim Öffnen der Ventile 5 und 6 höher ist als der Druck in der Permeatleitung. Nach Wahl der Betriebsphase Desinfektion fließt Permeat über den Anschluss 10 in das Dialysegerät. Die Ventile 21 und 22 sind geöffnet. Das Ventil 16 ist geschlossen und das Ventil 17 geöffnet. Der Fließdruck wird im Drucksensor 20 gemessen und mit dem gemessenen Druckwert hinter dem Ventil 21 verglichen. Liegen beide Werte im Toleranzfeld, wird der Desinfektionsvorgang fortgesetzt. Ist der Druck am Druck-Sensor 20 im Adapter kleiner als der notwendige Fließdruck im Dialysegerät, werden die Ventile 5 und 6 wieder geschlossen. Das Dialysegerät zeigt Wassermangelalarm an. Meldet der Druck-Sensor 20 einen genügend hohen Druck, bleiben die Ventile 5 und 6 geöffnet und Permeat kann für die Desinfektion weiter in das Dialysegerät einströmen. Hierbei fließt das Permeat nur über den Anschluss 10 und die Ventile 21 und 22. Während der Desinfektion zirkuliert die Desinfektionsflüssigkeit über das Ventil 17. In definierten Abständen wird das Ventil 17 geschlossen und das Ventil 16 geöffnet. Somit kann auch Desinfektionsmittel über die Kupplung 13 zum Adapter fließen und über den Anschluss 10 wieder zurück in das Vorlaufgefäß. Dieser Vorgang wiederholt sich in der Phase Desinfektion mehrmals. Somit ist die Permeat-Zuleitung zum Dialysegerät und der Leitungsabschnitt für Permeat im Dialysegerät mit in die Desinfektion eingebunden.

Am Ende der Desinfektion öffnet sich ein Ventil 23 (Figur 2) und es erfolgt die Freispülung des Dialysegerätes vom Desinfektionsmittel. Für diesen Vorgang muss Permeat in das Dialysegerät fließen. Hierfür müssen sich die Ventile 5 und 6 im Adapter öffnen. Dies erfolgt nur dann, wenn der Druck-Sensor 20 am Permeatanschluss des Adapters einen vorgegebenen Mindestdruck erfasst, der sicher über dem Druck im Dialysegerät liegen muss. Ist dies der Fall, werden die Ventile 5 und 6 geöffnet. Permeat fließt dann durch wechselseitiges Öffnen und Schließen der Ventile 16 und 17 über die Anschlüsse 10 und 13 in das Dialysegerät. Hierdurch ist sichergestellt, dass die Permeatzuleitungen zum Dialysegerät frei gespült werden. Das Dialysegerät kann nach Abschluss der Freispülung wieder in den Betriebszustand der Dialyse gebracht werden.

## Patentansprüche

1. Medizintechnisches Gerät, das an eine Wasserleitung zur Zufuhr von Wasser oder aufbereitetem Wasser in das medizintechnische Gerät anschließbar ist,
**enthaltend**
einen Adapter, der einen ersten Zuleitungsabschnitt (4) mit einem endseitigen Kupplungsteil (1) zur Verbindung mit der Wasserleitung (3), mit einem nachfolgenden Drucksensor (20) und mit zwei nachfolgenden, hintereinander angeordneten Absperrventilen (5,6) und zwei zweite Zuleitungsabschnitte (8,9) aufweist, die mittels eines Verzweigungsteils (7) von dem ersten Zuleitungsabschnitt (4) abzweigen, wobei einer der zweiten Zuleitungsabschnitte (8) über einen Anschluss (10) mit der Permeateinlassleitung (11) des medizintechnischen Gerätes verbunden ist, die in einen Vorlaufbehälter (12) einmündet, und der andere der zweiten Zuleitungsabschnitte (9) über einen Anschluss (13) und ein Absperrventil (16) mit einer anderen Flüssigkeitsleitung (15) des medizintechnischen Gerätes verbunden ist, in die ein Absperrventil (17) eingeschaltet ist und die ebenfalls in den Vorlaufbehälter (12) einmündet, wobei die andere Flüssigkeitsleitung (15) bei einem Desinfektionsvorgang von DesinfektionsFlüssigkeit durchströmbar ist und die Absperrventile (16 und 17) dabei wechselweise schaltbar sind, so dass Desinfektionsflüssigkeit abwechselnd durch die andere Flüssigkeitsleitung (15) in den Vorlaufbehälter (12) und durch die zweiten Zuleitungsabschnitte (8, 9) und die Permeateinlassleitung (11) in den Vorlaufbehälter (12) zirkuliert.

2. Medizintechnisches Gerät nach Anspruch 1,
**dadurch gekennzeichnet,**
**dass** der andere der zweiten Zuleitungsabschnitte (9) des Adapters über den Anschluss (13) an ein Zwischenleitungsstück (14) des medizintechnischen Gerätes angeschlossen ist, das mit der anderen Leitung (15) des medizintechnischen Gerätes verbunden ist.

3. Medizintechnisches Gerät nach Anspruch 2,
**dadurch gekennzeichnet,**
**dass** in dem Zwischenleitungsstück (14) das Absperrventil (16) angeordnet ist.

4. Medizintechnisches Gerät nach Anspruch 1,
**dadurch gekennzeichnet,**
**dass** das Kupplungsteil (1) Bestandteil eines Gehäuses ist, in dem die beiden Absperrventile (5,6) untergebracht sind.

5. Medizintechnisches Gerät nach einem der Ansprüche 1 bis 4,
**dadurch gekennzeichnet,**
**dass** die Absperrventile (5,6) Magnetventile sind, die über das Gerät angesteuert werden und auch ihre Stromversorgung aus dem Gerät erhalten.

6. Medizintechnisches Gerät nach Anspruch 5,
**dadurch gekennzeichnet,**
**dass** die Magnetventile (5,6) im stromlosen Zustand geschlossen sind.

7. Medizintechnisches Gerät nach einem der Ansprüche 1 bis 6,
**dadurch gekennzeichnet,**
**dass** Mittel zur automatischen Überprüfung der Stellung der beiden Ventile (5,6) vor jeder Behandlung und Desinfektion vorgesehen sind.

8. Medizintechnisches Gerät nach einem der Ansprüche 1 bis 7,
**dadurch gekennzeichnet,**
**dass** das medizintechnische Gerät ein Dialysegerät ist.

## Claims

1. A medical apparatus, which is connectable to a water conduit for the supply of water or purified water into the medical apparatus, including an adaptor, which includes a first feedline section (4) with a coupling member (1) at the end for connection to the water conduit (3), a subsequent pressure sensor (20) and two subsequent shut off valves (5, 6) and two second feedline sections (8, 9), which branch off from the first feedline section (4) by means of a branch member (7), wherein one of the second feedline sections (8) is connected via a connector (10) to the permeate inlet conduit (11) of the medical device, which communicates with a supply container (12) and the other of the second feedline sections (9) is connected via a connector (13) and a shut off valve (16) to another liquid conduit (15) of the medical device, connected into which is a shut off valve (17) and which also communicates with the supply container (12), wherein disinfection liquid may flow through the other liquid conduit (15) in a disinfection process and the shutoff valves (16 and 17) are switchable alternately so that the disinfection liquid circulates alternately through the other liquid conduit (15) into the supply container (12) and through the second feedline sections (8, 9) and the permeate inlet conduit (11) into the supply container (12).

2. A medical apparatus as claimed in claim 1, **characterised in that** the other of the second feedline sections (9) of the adaptor is connected via the connector (13) to an intermediate conduit member (14) of the medical device, which is connected to the other conduit (15) of the medical device.

3. A medical apparatus as claimed in claim 2, **characterised in that** the shut off valve (16) is arranged in the intermediate conduit member (14).

4. A medical apparatus as claimed in claim 1, **characterised in that** the coupling member (1) is a component of a housing, in which the two shut off valves (5, 6) are accommodated.

5. A medical apparatus as claimed in one of claims 1 to 4, **characterised in that** the shut off valves (5, 6) are magnetic valves, which are controlled by the apparatus and also obtain their power supply from the apparatus.

6. A medical apparatus as claimed in claim 5, **characterised in that** the magnetic valves (5, 6) are closed in the unpowered state.

7. A medical apparatus as claimed in one of claims 1 to 6, **characterised in that** means are provided for automatically checking the position of the two valves (5, 6) before each treatment and disinfection.

8. A medical apparatus as claimed in one of claims 1 to 7, **characterised in that** the medical apparatus is a dialysis apparatus.

## Revendications

1. Appareil de technique médicale qui peut être raccordé à une conduite d'eau pour l'acheminement d'eau ou d'eau conditionnée dans l'appareil de technique médicale,
contenant
un adaptateur qui présente un premier segment d'acheminement (4) comportant une partie de couplage terminale (1) destinée à être reliée à la conduite d'eau (3) comportant un capteur de pression consécutif (20) et deux robinets (5, 6) disposés consécutivement l'un après l'autre, et deux deuxièmes segments d'acheminement (8, 9) qui dérivent au moyen d'une partie de ramification (7) du premier segment d'acheminement (4), l'un des deuxièmes segments d'acheminement (8) étant relié par un raccordement (10) à la conduite d'entrée du perméat (11) de l'appareil de technique médicale qui débouche dans un réservoir d'alimentation (12) et l'autre des deuxièmes segments d'acheminement (9) étant relié par un raccordement (13) et un robinet (16) à une autre conduite de liquide (15) de l'appareil de technique médicale dans laquelle un robinet (17) est intercalé et qui débouche éventuellement dans le réservoir d'alimentation (12), l'autre conduite de liquide (15) pouvant être parcourue, dans un processus de désinfection, par un liquide de désinfection et les robinets (16 et 17) pouvant être commutés alternativement de sorte que le liquide de désinfection circule alternativement dans l'autre conduite de liquide (15) dans le réservoir d'alimentation (12) et dans les deuxièmes segments d'acheminement (8, 9) et la conduite d'entrée de perméat (11) dans le réservoir d'alimentation (12).

2. Appareil de technique médicale selon la revendication 1,
**caractérisé en ce que**
l'autre deuxième segment d'acheminement (9) de l'adaptateur est raccordé par le raccordement (13) à une pièce d'acheminement intermédiaire (14) de l'appareil médical qui est relié à l'autre conduite (15) de l'appareil de technique médicale.

3. Appareil de technique médicale selon la revendication 2,
**caractérisé en ce que**
le robinet (16) est disposé dans la pièce d'acheminement intermédiaire (14).

4. Dispositif de technique médicale selon la revendication 1,
**caractérisé en ce que**
la partie de couplage (1) est un composant d'un boîtier dans lequel les deux robinets (5, 6) sont placés.

5. Appareil de technique médicale selon l'une des revendications 1 à 4,
**caractérisé en ce que**
les robinets (5, 6) sont des soupapes magnétiques qui sont commandées par l'appareil et tirent également leur alimentation électrique de l'appareil.

6. Appareil de technique médicale selon la revendication 5,
**caractérisé en ce que**
les soupapes magnétiques (5, 6) sont fermées à l'état hors tension.

7. Appareil de technique médicale selon l'une des revendications 1 à 6,
**caractérisé en ce que**
le moyen de contrôle automatique de la position des deux soupapes (5, 6) est prévu avant chaque traitement et désinfection.

8. Appareil de technique médicale selon l'une des revendications 1 à 7,
**caractérisé en ce que**
l'appareil de technique médicale est un appareil de dialyse.
